(19) **Europäisches Patentamt / European Patent Office / Office européen des brevets**

(11) **EP 2 881 874 B1**

(12) **EUROPEAN PATENT SPECIFICATION**

(45) Date of publication and mention
of the grant of the patent:
**28.08.2019 Bulletin 2019/35**

(51) Int Cl.:
***G16C 20/30*** *(2019.01)*

(21) Application number: **14196003.9**

(22) Date of filing: **03.12.2014**

(54) **System and method for searching for new material**

System und Verfahren zur Suche nach neuem Material

Système et procédé de recherche de nouveau matériau

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2013 KR 20130149495**

(43) Date of publication of application:
**10.06.2015 Bulletin 2015/24**

(73) Proprietor: **Samsung Electronics Co., Ltd.
Gyeonggi-do 443-742 (KR)**

(72) Inventors:
 • **Yoo, Jiho**
  **443-803 Gyeonggi-do (KR)**
 • **Hwang, Sangheum**
  **443-803 Gyeonggi-do (KR)**
 • **Kim, Sungjin**
  **443-803 Gyeonggi-do (KR)**
 • **Lee, Sanghyun**
  **443-803 Gyeonggi-do (KR)**
 • **Lee, Chanhee**
  **443-803 Gyeonggi-do (KR)**

(74) Representative: **Elkington and Fife LLP
Prospect House
8 Pembroke Road
Sevenoaks, Kent TN13 1XR (GB)**

(56) References cited:
**US-A1- 2009 157 369**

 • ZUBKOV V. G. ET AL: "Synthesis and crystal structure of Ln2M<2+>Ge4O12, Ln=rare-earth element or Y; M=Ca, Mn, Zn", JOURNAL OF SOLID STATE CHEMISTRY, ORLANDO, FL, US, vol. 183, no. 5, 1 May 2010 (2010-05-01), pages 1186-1193, XP027039718, ISSN: 0022-4596 [retrieved on 2010-03-20]
 • FILIZ M. ET AL: "EXTRACTION OF TITANIUM(IV) FROM AQUEOUS HYDROCHLORIC ACID SOLUTIONS INTO ALAMINE 336-M-XYLENE MIXTURES", CHEMICAL ENGINEERING COMMUNICATIONS, vol. 193, no. 9, 30 August 2006 (2006-08-30), pages 1127-1141, XP055180427, ISSN: 0098-6445, DOI: 10.1080/00986440500354457

**Description**

BACKGROUND

FIELD OF THE INVENTION

Example embodiments relate to a system and method for searching for a new material, and/or to a system and method in which a new material is searched for on the basis of predicted exchange tendency data of ions constituting a crystal of the material.

BACKGROUND OF THE INVENTION

To develop a new material, new materials may be generally searched for through a method of generating a new material candidate group by using already known structures or composition data of materials and verifying the generated new material candidate group.

Meanwhile, by substituting an ion constituting an existing material with another ion, a candidate group for a new material may be generated. At this time, to substitute the ion constituting the existing material with another ion, exchange tendency data of the ion is used.

According to related art, only known exchange tendency data of ions is used. Therefore, when not enough existing data is available, or when most data is concentrated only on particular ions, it is typically difficult to accurately search for a new material.

US2009/0157369 describes a high throughput search engine for materials using quantum simulations.

SUMMARY OF THE INVENTION

Example embodiments relate to a system and method for searching for a new material in which an unknown ion substitution tendency may be predicted to ensure the diversity of new material candidate groups and accurately predict the probability of substitution of the new material.

Additional example embodiments will be set forth in part in the description which follows and, in part, will be apparent from the description, or may be learned by practice of the example embodiments.

According to an aspect, there is provided a method of searching for a new material according to claim 1.

The calculating of the ion property matrix U includes calculating the ion property matrix U for minimizing.

According to an example embodiment, the calculating of the ion property matrix U may further include assigning a weight to $X - UU^T$ to exclude data indicating that a substitution tendency is unknown from elements of the substitution tendency matrix X during a process of the calculating.

According to an example embodiment, the method may further include acquiring a prior information matrix Y including prior information data, and the calculating of the ion property matrix U may include calculating the ion property matrix U based on the substitution tendency matrix X and the prior information matrix Y.

According to an example embodiment, the calculating of the ion property matrix U may include calculating the ion property matrix U by applying a matrix co-factorization model, which causes the ion property matrix U to be included in the substitution tendency matrix X and the prior information matrix Y in common, to the substitution tendency matrix X and the prior information matrix Y.

According to an example embodiment, the calculating of the ion property matrix U may include calculating the ion property matrix U under a constraint condition that element values of the ion property matrix U are not negative numbers.

According to an example embodiment, the prior information data may include at least one of oxidation number data of the ions and radius data of the ions.

According to an example embodiment, the acquiring of the prior information matrix Y may include determining element values of the prior information matrix Y based on a distribution of the prior information data.

According to an example embodiment, the method may further include generating the new predicted crystal structures in decreasing order of the probabilities of substitution of the new crystal structures.

According to an aspect, there is provided a system for searching for a new material according to claim 6.

The substitution tendency extractor calculates the ion property matrix U for minimizing $X - UU^T$.

According to an example embodiment, the substitution tendency extractor may assign a weight to $X - UU^T$ to exclude data indicating that a substitution tendency is unknown from elements of the substitution tendency matrix X during a process of the calculation.

According to an example embodiment, the system may further include a prior information model builder which calculates a prior information matrix Y including prior information data, and the substitution tendency extractor may calculate the ion property matrix U based on the substitution tendency matrix X and the prior information matrix Y.

According to an example embodiment, the substitution tendency extractor may calculate the ion property matrix U by applying a matrix co-factorization model, which causes the ion property matrix U to be included in the substitution tendency matrix X and the prior information matrix Y in common, to the substitution tendency matrix X and the prior information matrix Y.

According to an example embodiment, the substitution tendency extractor may calculate the ion property matrix U under a constraint condition that element values of the ion property matrix U are not negative numbers.

According to an example embodiment, the prior information model builder may determine element values of the prior information matrix Y based on a distribution of the prior information data.

According to an example embodiment, the system may further include a new crystal or molecular structure predictor configured to generate new predicted crystal structures in decreasing order of the probabilities of substitution of the new crystal structures.

## BRIEF DESCRIPTION OF THE DRAWINGS

These and/or other example embodiments will become apparent and more readily appreciated from the following description, taken in conjunction with the accompanying drawings in which:

FIG. 1 is a block diagram showing a constitution of a system for searching for a new material according to an example embodiment;
FIGS. 2 and 3 are diagrams illustrating an ion exchange tendency according to an example embodiment;
FIG. 4 is a flowchart of a method of searching for a new material;
FIG. 5 is a flowchart of a method of searching for a new material according to another described aspect;
FIG. 6 is a diagram illustrating a method of determining the number of ionic properties according to a described aspect;
FIG. 7 is a diagram showing a distribution of prior information according to an example embodiment; and
FIG. 8 shows prior information models for some elements according to an example embodiment.

## DETAILED DESCRIPTION

Reference will now be made in detail to example embodiments illustrated in the accompanying drawings, wherein like reference numerals refer to like elements throughout. In this regard, the example embodiments may have different forms and should not be construed as being limited to the descriptions set forth herein. Accordingly, the example embodiments are merely described below, by referring to the figures. Expressions such as "at least one of," when preceding a list of elements, modify the entire list of elements and do not modify the individual elements of the list.

It will be understood that when an element is referred to as being "on," "connected" or "coupled" to another element, it can be directly on, connected or coupled to the other element or intervening elements may be present. In contrast, when an element is referred to as being "directly on," "directly connected" or "directly coupled" to another element, there are no intervening elements present. As used herein the term "and/or" includes any and all combinations of one or more of the associated listed items. Further, it will be understood that when a layer is referred to as being "under" another layer, it can be directly under or one or more intervening layers may also be present. In addition, it will also be understood that when a layer is referred to as being "between" two layers, it can be the only layer between the two layers, or one or more intervening layers may also be present.

It will be understood that, although the terms "first", "second", etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of example embodiments.

In the drawing figures, the dimensions of layers and regions may be exaggerated for clarity of illustration. Like reference numerals refer to like elements throughout. The same reference numbers indicate the same components throughout the specification.

Spatially relative terms, such as "beneath," "below," "lower," "above," "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

The terminology used herein is for the purpose of describing particular embodiments only and is not intended to be limiting of example embodiments. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Example embodiments are described herein with reference to cross-sectional illustrations that are schematic illustrations of idealized embodiments (and intermediate structures) of example embodiments. As such, variations from the shapes of the illustrations as a result, for example, of manufacturing techniques and/or tolerances, are to be expected. Thus, example embodiments should not be construed as limited to the particular shapes of regions illustrated herein but are to include deviations in shapes that result, for example, from manufacturing. For example, an implanted region illustrated as a rectangle will, typically, have rounded or curved features and/or a gradient of implant concentration at its edges rather than a binary change from implanted to non-implanted region. Likewise, a buried region formed by implantation may result in some implantation in the region between the buried region and the surface through which the implantation takes place. Thus, the regions illustrated in the figures are schematic in nature and their shapes are not intended to illustrate the actual shape of a region of a device and are not intended to limit the scope of example embodiments.

Unless otherwise defined, all terms (including technical and scientific terms) used herein have the same meaning as commonly understood by one of ordinary skill in the art to which example embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein. As used herein, expressions such as "at least one of," when preceding a list of elements, modify the entire list of elements and do not modify the individual elements of the list.

Although corresponding plan views and/or perspective views of some cross-sectional view(s) may not be shown, the cross-sectional view(s) of device structures illustrated herein provide support for a plurality of device structures that extend along two different directions as would be illustrated in a plan view, and/or in three different directions as would be illustrated in a perspective view. The two different directions may or may not be orthogonal to each other. The three different directions may include a third direction that may be orthogonal to the two different directions. The plurality of device structures may be integrated in a same electronic device. For example, when a device structure (e.g., a memory cell structure or a transistor structure) is illustrated in a cross-sectional view, an electronic device may include a plurality of the device structures (e.g., memory cell structures or transistor structures), as would be illustrated by a plan view of the electronic device. The plurality of device structures may be arranged in an array and/or in a two-dimensional pattern.

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain example embodiments of the present description.

In this specification, description will be made by taking ions as an example for convenience, but the example embodiments may be likewise applied to neutral atoms.

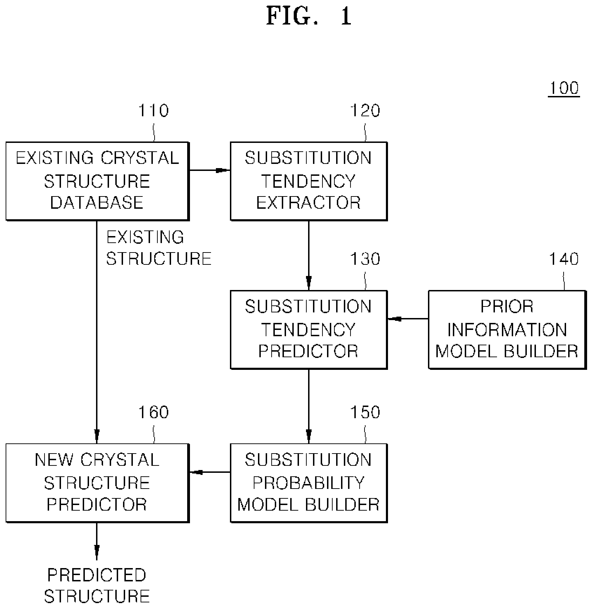

FIG. 1 is a block diagram showing a system 100 for searching for a new material according to an example embodiment.

Referring to FIG. 1, the system 100 for searching for a new material may include an existing crystal or molecular structure database 110, a substitution tendency extractor 120, a substitution tendency predictor 130, a prior information model builder 140, a substitution probability model builder 150, and a new crystal structure predictor 160.

The existing crystal or molecular structure database 110 may be a database including information on the crystal structures of already known materials.

According to an example embodiment, the information on the crystal structures may include the sizes of unit cells of the crystal structures, information on the relative positions of respective atoms included in the crystal structures in the unit cells, and so on.

The substitution tendency extractor 120 may include a processor to group together materials having the same structure, on the basis of the information of an existing crystal structure database, and to analyze the materials which are grouped together based on their same structures and have different ions at the same structural position, thereby extracting or determining substitution tendencies of the ions. For example, the materials may include a first material and a second material. Here, the first material has a crystal structure C1, and the second material has a crystal structure C2. Also, the crystal structure C1 is the same as the crystal structure C2, and a position P1 on the crystal structure C1 is the same as a position P2 on the crystal structure C2. However, an ion I1 located at the position P1 is different than an ion I2 located at the position P2.

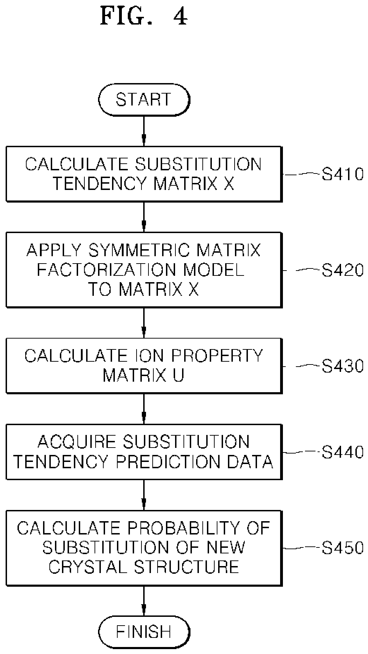

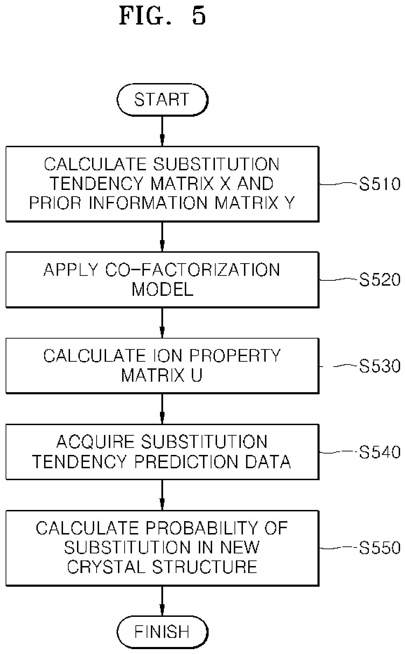

According to at least one example embodiment, the processor may be an arithmetic logic unit, a digital signal processor, a microcomputer, a field programmable array, a programmable logic unit, a microprocessor or any other device capable of responding to and executing instructions in a defined manner such that the processor is programmed with instructions that configure the processing device as a special purpose computer to perform the operations illustrated in Figs. 4 and 5. The instructions may be stored on a non-transitory computer readable medium. Examples of non-transitory computer-readable media include magnetic media such as hard disks, floppy disks, and magnetic tape; optical media such as CD ROM discs and DVDs; magneto-optical media such as optical discs; and hardware devices that are specially configured to store and perform program instructions, such as read-only memory (ROM), random access memory (RAM), flash memory, and the like. The non-transitory computer-readable media may also be a distributed network, so that the program instructions are stored and executed in a distributed fashion.

For example, the substitution tendency extractor 120 may collect the frequency that two ions (or elements) are present at the same position in two materials having the same structure, determine that the higher the frequency, the higher the probability of substitution, that is, the probability that two ions will be substituted for each other, and construct a substitution tendency matrix X according to the probability of substitution. The substitution tendency matrix X is described in detail later.

The prior information model builder 140 may build a prior information model by using known prior information data. For example, the prior information data may include at least one of oxidation number data of ions and radius data of the ions. In particular, the prior information model builder 140 may build a prior information matrix Y by using a distribution of the prior information data. This is described in detail later.

The substitution tendency predictor 130 calculates substitution tendency prediction data by using the substitution tendency matrix X and the prior information matrix Y. Here, the substitution tendency prediction data is a value which denotes an unknown ion substitution tendency.

The substitution probability model builder 150 may calculate the probability of substitution on the basis of the substitution tendency prediction data calculated by the substitution tendency predictor 130.

For example, the probability of substitution between a material A and a material B may be calculated by using the following probability model.

[Expression 1]

$$ p(A,B) = \frac{1}{Z} \prod_{i=1}^{n} \exp\left( \lambda_{x^{(i)} y^{(i)}} \right), $$

In 1/z(.), z is a value for causing the value of (.) function to satisfy a probability condition (that the sum of probability values is 1), and may be calculated by summing (.) values for all possible combinations of A and B. Also, on the basis of the substitution tendency prediction data, a parameter λ may be obtained by repeatedly applying the following expression:

[Expression 2]

$$ \lambda_{cd}^{(t+1)} = \log\left( \frac{R_{cd}}{R_S - R_{cd}} \right) + \log\left( \sum_{(a,b)\in P,\ (c,d)} \exp\left( \lambda_{ab}^{(t)} \right) \right). $$

Here, $\lambda_{cd}$ is a parameter obtained where a c ion and d ion are substituted for each other, Rs denotes the sum of all substitution tendency prediction data calculated as UU', and Rcd denotes substitution tendency prediction data between the c ion and d ion.

The new crystal structure predictor 160 may obtain an existing crystal structure from the existing crystal structure database 110 on the basis of the calculated probability of substitution, and generate a new crystal structure B of new composition by substituting an ion B for an ion A included in the obtained existing crystal structure.

For example, the new crystal structure predictor 160 may generate new crystal structures in order of decreasing probability of substitution. Alternatively, the new crystal structure predictor 160 may generate a new crystal structure only when the probability of substitution is a previously set or a desired value or more.

A method of acquiring substitution tendency prediction data is described in detail below.

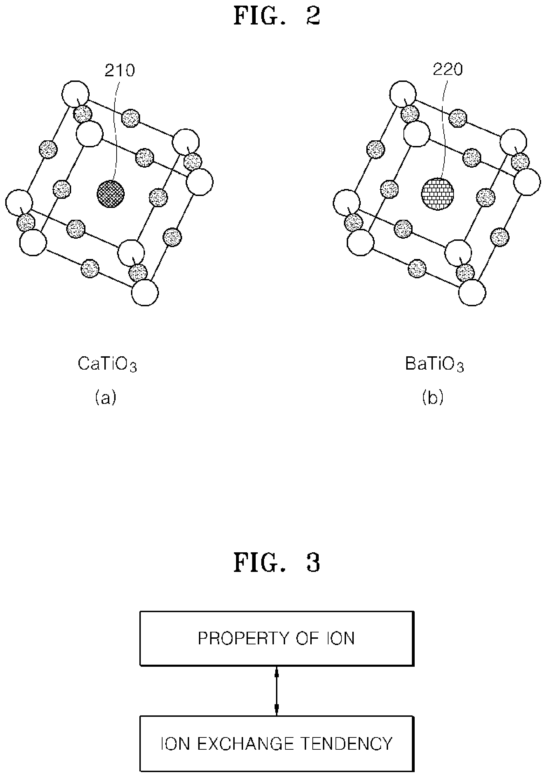

FIGS. 2 and 3 are diagrams illustrating an ion exchange tendency.

An ion exchange tendency may be a value which denotes the probability that two ions can be substituted for each other. For example, calcium titanate ($CaTiO_3$) of FIG. 2(A) and barium titanium oxide ($BaTiO_3$) of FIG. 2(B) are materials having the same or equivalent structure, and a calcium ion ($Ca^{2+}$) 210 and a barium ion ($Ba^{2+}$) 220 are present at the same or equivalent position in the two materials, respectively.

As described above, it is possible to assume that different ions present at the same or equivalent position in two materials having the same structure may be substituted for each other. In other words, it is possible to assume that the calcium ion ($Ca^{2+}$) 210 and the barium ion ($Ba^{2+}$) 220 may be substituted for each other.

Also, it is possible to collect the frequency at which two ions are present at the same or equivalent position in two materials having the same or equivalent structure, and determine that the higher the frequency, the higher the probability of substitution between the two ions. As the probability of substitution increases, the amount of substitution tendency data may be larger.

As illustrated in FIG. 3, such an ion substitution tendency may be related to features of the ions. For example, when an ion A and an ion B have similar or the same properties, they may have similar or the same substitution tendencies. Also, when the ion A and the ion B have similar or the same substitution tendencies, they may have similar or the same properties.

Therefore, prediction of an unknown substitution tendency may be performed through a method of 1) extracting properties of an ion which are not directly observed from known substitution tendencies, and 2) predicting the unknown ion substitution tendency from the extracted properties of the ion.

FIG. 4 is a flowchart of a method of searching for a new material.

Referring to FIG. 4, the system 100 for searching for a new material may calculate a substitution tendency matrix X (operation 410). Here, the substitution tendency matrix X may include known substitution tendency data between ions. The substitution tendency matrix X is described in detail below.

<<Expression of substitution tendency vector x>>

A substitution tendency of an ion may be expressed in the form of a vector. When substitution tendencies for N ions are expressed, a vector x may have N components, and each of the N components may be a value denoting a substitution tendency of any one ion for one of the N ions.

For example, when $x_i$ is presumed to be a substitution tendency vector of an i-th ion among the N ions, a first component $x(1i)$ of $x_i$ may be a value denoting a substitution tendency of the i-th ion for a first ion, and a second component $x(2i)$ of $x_i$ may be a value denoting a substitution tendency of the i-th ion for a second ion.

When a substitution tendency of the i-th ion for a j-th ion is unknown, a j-th component $x(ji)$ of $x_i$ may be set to 0.

The substitution tendency vector $x_i$ may be expressed as a linear combination of several ion property vectors ($u_1$, $u_2$, ..., and $u_R$) as follows:

[Expression 3]

$$\mathbf{x}_i \approx a_{1i} \times \mathbf{u}_1 + a_{2i} \times \mathbf{u}_2 + a_{3i} \times \mathbf{u}_3 + \cdots + a_{Ri} \times \mathbf{u}_R.$$

Here, properties of the ion may be classified into R types, and each $\mathbf{u}_j$ is a vector denoting a j-th property among the R property types. Meanwhile, $\mathbf{u}_j$ may have the same size as $\mathbf{x}_i$. In other words, $\mathbf{u}_j$ may have N components.

Also, each value of $a_{ji}$ may denote how important the j-th ion property vector is in expressing a substitution tendency of the i-th ion. In other words, each value of $a_{ji}$ may be a weight indicating how much of the j-th property the i-th ion has.

Meanwhile, when a substitution tendency vector is expressed in the form of Expression 3, the substitution tendency vector may be expressed with a number of ion property vectors that is smaller than a number of existing ion property vectors. In other words, when the number of existing ion property vectors is Y, the substitution tendency vector may be expressed with only R ion property vectors obtained by condensing the properties of the ion.

These ion property vectors may represent properties of the ion, which are not acquired from existing property data, in a simple form which is easy to recognize.

Meanwhile, the substitution tendency vector $x_i$ of the i-th ion may be expressed as follows:

[Expression 4]

$$\mathbf{x}_i \approx \mathbf{U} \mathbf{a}_i.$$

An ion property matrix U includes ion property vectors $u_1$, $u_2$, ..., and $u_R$ of Expression 3. Thus, for an ion property vector having N components, the size of the ion property matrix U may be N*R.

$a_i$ is a weight vector. The weight vector $a_i$ has R components, which are the weights $a_{1i}$, $a_{2i}$, ..., and $a_{Ri}$ of Expression 3.

Meanwhile, substitution tendency vectors (x1, x2, ..., and xN) of the N ions may be expressed as one substitution tendency matrix X. For example, each column of the substitution tendency matrix X may be configured to represent a substitution tendency vector of an ion, and thus an i-th column of the substitution tendency matrix X may represent the substitution tendency vector $x_i$ of the i-th ion.

When a substitution tendency vector of an ion has N components, the size of the matrix X may be N*N, and the matrix X may be a square matrix.

<<Basic matrix factorization model>>

By applying a basic matrix factorization model to the substitution tendency matrix X, the substitution tendency matrix X may be expressed as follows:

[Expression 5]

$$\mathbf{X} \approx \mathbf{U}\mathbf{A}^{\mathrm{T}}.$$

Here, U is an ion property matrix, and A is a weight matrix including weight vectors for the N ions. As described above, for weight vector $a_i$ having R components, the size of the weight matrix A may be N*R.

$\mathbf{A}^{\mathrm{T}}$ denotes a transpose matrix of the weight matrix A, which is obtained by interchanging rows and columns of the weight matrix A.

The substitution tendency matrix X denotes the substitution tendency between two ions. In the substitution tendency matrix X, (i, j) element (the substitution tendency between the i-th ion and the j-th ion) and (j, i) element (the substitution tendency between the j-th ion and the i-th ion) have the same value.

Therefore, the substitution tendency matrix X has the form of a symmetric matrix.

In this case, when Expression 5 is applied as it is, it may be difficult to represent the symmetry of the substitution tendency matrix X. In other words, the substitution tendency of the i-th ion for the j-th ion may become different from the substitution tendency of the j-th ion for the i-th ion.

Consequently, it is necessary to use an expression for maintaining the symmetry of the substitution tendency matrix X, and thus a symmetric matrix factorization model may be applied to the substitution tendency matrix X (operation 420).

<<Symmetric matrix factorization model>>

As described above, in the substitution tendency matrix X, both rows and columns denote ions and are symmetrical to each other. Also, in the ion property matrix U, rows denote ions, and columns denote properties of the ions.

On the basis of the characteristics described above, the substitution tendency matrix X may be expressed by using the ion property matrix U as follows:

[Expression 6]

$$\mathbf{X} \approx \mathbf{U}\mathbf{U}^{\mathrm{T}}.$$

When Expression 6 is used, it is possible to effectively determine the ion property matrix U while maintaining the symmetry of the substitution tendency matrix X.

The system 100 for searching for a new material may calculate the ion property matrix U by using an expression (operation 430). This is described in detail below.

<<Learning of matrix factorization model>>

As described above, when a matrix factorization model is determined as Expression 6 and the substitution tendency matrix X is given, optimization may be performed to minimize a difference $\mathbf{X} - \mathbf{U}\mathbf{U}^{\mathrm{T}}$ between the substitution tendency matrix X and the matrix factorization model $\mathbf{U}\mathbf{U}^{\mathrm{T}}$ so that the ion property matrix U is determined.

By using the Frobenius norm $\left( \|\mathbf{A}\|_F = \sqrt{\sum a_{ij}^2} \right)$ of a matrix, the above description may be expressed as the following optimization problem:

[Expression 7]

$$\mathrm{argmin}_U \left\| \mathbf{X} - \mathbf{U}\mathbf{U}^T \right\|_F.$$

When the substitution tendency matrix X is given, the ion property matrix U of Expression 7 may be calculated.

Meanwhile, according to an example embodiment, the above Expression 7 may be expressed as follows:

[Expression 8]

$$\mathrm{argmin}_U \left\| \mathbf{W} \odot (\mathbf{X} - \mathbf{U}\mathbf{U}^T) \right\|_F.$$

Here, W is a weight matrix, serving to exclude data indicating that the corresponding ion substitution tendency is unknown, that is, an element represented as 0 among elements of the substitution tendency matrix X.

For example, when the substitution tendency of the i-th ion for the j-th ion is unknown, (i, j) element and (j, i) element of the substitution tendency matrix X may be represented as 0. Accordingly, the weight matrix W may exclude data indicating that the corresponding ion substitution tendency is unknown so that the ion property matrix U may be calculated.

The weight matrix W has the same size as the substitution tendency matrix X. An element of the weight matrix W may be set to 1 when the corresponding element of the substitution tendency matrix X has an observed value (in the case of a known ion substitution tendency), and to 0 when the corresponding element does not have an observed value (in the case of an unknown ion substitution tendency).

For example, when the substitution tendency of the i-th ion for the j-th ion is unknown and (i, j) element of the substitution tendency matrix X is 0, (i, j) element of the weight matrix W may also be set to 0.

Meanwhile, the operator $\odot$ of Expression 8 denotes multiplication of elements of matrices. For example, according to C = A$\odot$B, (i, j) element of a matrix C may be represented as a product ($c_{ij} = a_{ij} \times b_{ij}$) of (i, j) element of a matrix A and (i, j) element of a matrix B, sometimes referred to as the Hadamard product.

Therefore, Expression 8 makes it possible to prevent or reduce having an unobserved value (substitution tendency data indicating that the corresponding ion substitution tendency is unknown) among the elements of the substitution tendency matrix X from being used in an optimization process of calculating the ion property matrix U.

Meanwhile, by using the calculated ion property matrix U, the system 100 for searching for a new material may calculate a reconstructed substitution tendency matrix X' as follows, and thus acquire substitution tendency prediction data (operation 440).

[Expression 9]

$$\mathbf{X}' = \mathbf{U}\mathbf{U}^T$$

The reconstructed substitution tendency matrix X' may include a predicted value (substitution tendency prediction data) for an ion substitution tendency which is not observed in the substitution tendency matrix X.

In other words, an element represented as 0 in the substitution tendency matrix X may be represented as a predicted value (substitution tendency prediction data) of an ion substitution tendency other than 0 in the reconstructed ion exchange tendency matrix X'.

Therefore, it is possible to predict an unknown substitution tendency between two ions, according to at least one example embodiment.

As described in FIG. 1, the system 100 for searching for a new material may calculate the probability of substitution of a new crystal structure on the basis of the substitution tendency prediction data (operation 450).

FIG. 5 is a flowchart of a method of searching for a new material according to another described aspect.

The system 100 for searching for a new material may expand a model to use additional data (prior information data) as well as substitution tendency data (a substitution tendency matrix X) and calculate an ion property matrix U.

Therefore, the system 100 for searching for a new material may calculate the substitution tendency matrix X and a prior information matrix Y (operation 510).

Here, the prior information matrix Y represents additional properties of ions, and each row of the prior information matrix Y may consist of vectors including additional property data of one ion. When the number of ions is N and there are K additional properties, the prior information matrix Y may have a size of N*K.

Meanwhile, the prior information matrix Y is intended to calculate the ion property matrix U, and thus the system 100 for searching for a new material may apply a matrix co-factorization model to the prior information matrix Y as follows (operation 520):

[Expression 10]

$$\mathbf{Y} \approx \mathbf{U}\mathbf{V}^{\mathrm{T}}$$

In Expression 10, the ion property matrix U used in Expression 5 may be used as-is. A matrix V may be a matrix which represents weights for expressing the prior information matrix Y as properties of ions, or characteristics of additional information.

When some unobserved values are also in the additional information, weighted matrix factorization may be applied as in Expression 8, and then an optimization problem may be derived and expressed as follows:

[Expression 11]

$$\mathrm{argmin}_{\mathbf{U},\mathbf{V}}[\left\|\mathbf{W_x}\odot(\mathbf{X}-\mathbf{U}\mathbf{U}^{\mathrm{T}})\right\|_{\mathrm{F}} + \lambda\left\|\mathbf{W_y}\odot(\mathbf{Y}-\mathbf{U}\mathbf{V}^{\mathrm{T}})\right\|_{\mathrm{F}}].$$

Here, $\lambda$ is a parameter for balancing two matrix factorizations, and generally serves to compensate for a difference in size between the substitution tendency matrix X and the prior information matrix Y when the difference is large.

Meanwhile, the system 100 for searching for a new material may calculate the ion property matrix U by using Expression 11 (operation 530). This is described in detail below.

To solve the optimization problem shown in Expression 11, an algorithm or process may be used in which repeated optimization is performed to find an answer in stages.

Since there are two target matrices U and V, first, the matrix V is fixed at an arbitrary value, and only the matrix U may be optimized (first operation). Next, the matrix U is fixed at an arbitrary value, and only the matrix V may be optimized (second operation).

The first and second operations are repeatedly performed until the matrices U and V converge, and thus optimum matrices U and V may be calculated.

In the first operation, it is possible to calculate a gradient of an optimization objective function with respect to U and perform optimization while changing U in the corresponding direction.

For example, a gradient of an optimization objective function E of Expression 11 with respect to U may be expressed as the following expression:

[Expression 12]

$$\frac{\partial \mathbf{E}}{\partial \mathbf{U}} = -4(\mathbf{W_X}\odot\mathbf{X})\mathbf{U} + 4(\mathbf{W_X}\odot\mathbf{U}\mathbf{U}^{\mathrm{T}})\mathbf{U} - 2\lambda(\mathbf{W_Y}\odot\mathbf{Y})\mathbf{V} + 2\lambda(\mathbf{W_Y}\odot\mathbf{U}\mathbf{V}^{\mathrm{T}})\mathbf{V}$$

Accordingly, U may be calculated by using the following gradient descent method:

[Expression 13]

$$\mathbf{U} \leftarrow \mathbf{U} - \eta\frac{\partial \mathbf{E}}{\partial \mathbf{U}}.$$

Here, $\eta$ is a parameter for determining the speed of an algorithm or process. When the value of $\eta$ is excessively large, U may not converge to an arbitrary value, and when the value is small, the convergence speed of the algorithm may be low. Therefore, $\eta$ may be determined to be a value which causes no problems in the process of testing the algorithm.

In the second operation, it is possible to calculate a gradient of an optimization objective function with respect to V, and perform optimization while changing V in the direction corresponding to the gradient.

For example, a gradient of an optimization objective function E with respect to U may be expressed as the following expression:

[Expression 14]

$$\frac{\partial E}{\partial V} = -2\lambda(W_Y \odot Y)^T U + 2\lambda(W_Y \odot UV^T)^T U.$$

Likewise, V may be calculated by using the following gradient descent method:

[Expression 15]

$$V \leftarrow V - \eta \frac{\partial E}{\partial V}.$$

By repeatedly performing the first and second operations expressed above with respect to Expressions 14 and 15 until a variation in the value of the objective function E becomes a predetermined or alternatively desired value or less (until the variation converges to a predetermined or alternatively desired value), the ion property matrices U and V may be finally calculated.

<<Addition of constraint condition>>

According to at least one example embodiment, a constraint condition may be added to the ion property matrix U. For example, it is possible to add a constraint condition that the values of respective elements of the ion property matrix U may not be negative numbers. Accordingly, elements of the substitution tendency matrix X may not have negative values.

In order to obtain a result which satisfies such a constraint condition, it is possible to add a third operation of projecting the matrix U calculated by repeatedly performing the first and second operations onto an area in which the constraint condition is satisfied.

The third operation may be expressed as the following expression:

[Expression 16]

$$U \leftarrow f\left(U - \eta \frac{\partial E}{\partial U}\right).$$

Here, a function f(.) changes a given value into a value which is closest to the given value while satisfying the constraint condition. For example, when the constraint condition is that the values of the elements of the matrix U may not be negative numbers, f(.)is used to simply change negative values among the elements of the matrix U calculated in the first operation into 0.

As described above, the system 100 for searching for a new material may calculate the ion property matrix U by additionally using prior information data, and calculate a reconstructed substitution tendency matrix X' including substitution tendency prediction data by using the ion property matrix U (operation 540).

In addition, as described in FIG. 1, the system 100 for searching for a new material may calculate the probability of substitution of a new crystal structure on the basis of the substitution tendency prediction data (operation 550).

Description is made in detail below for a method of determining an ion property number R, which is the number of columns of the ion property matrix U, and a method of constructing the prior information matrix Y, according to various example embodiments.

<<Determination of ion property number>>

In a model for expressing the substitution tendency matrix X, such as Expression 3, the number of ion property vectors ($u_1$, $u_2$, ..., and $u_R$) may be defined to be R. Here, as the number of ion property vectors (the number of columns of the ion property matrix U) is increased, the properties of an ion may be expressed more precisely. However, noise included in the calculated ion property matrix U may increase, and a calculation time also increases.

Therefore, it may be advantageous to determine the number of ion property vectors so that the ion property vectors accurately reflect the main property data.

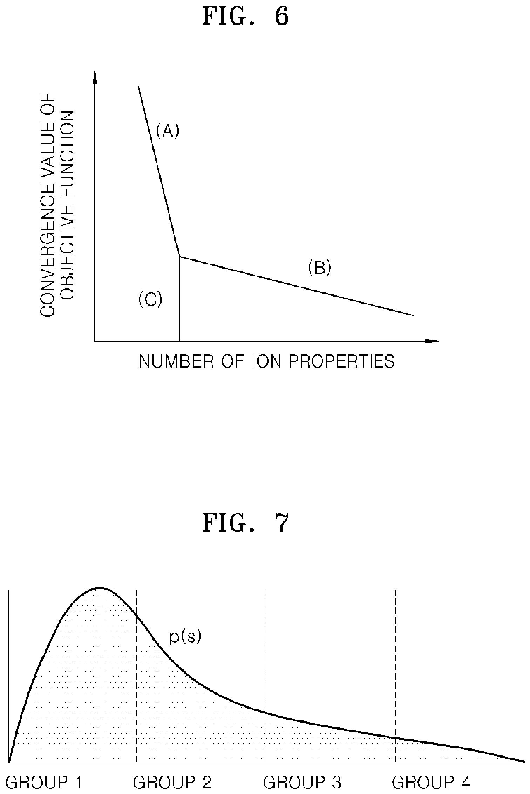

FIG. 6 is a graph illustrating what value an objective function value of an optimization problem converges to according to the ion property number R in at least one described aspect.

The objective function value is a difference between the substitution tendency matrix X and a factorization model for the substitution tendency matrix X.

Referring to the graph of FIG. 6, as the number of ion properties increases, it becomes possible to give a precise expression, and a convergence value of the objective function is reduced.

Referring to the graph of FIG. 6, in a section (A) where the number of ion properties is small, every time the number of ion properties increases by one, it becomes possible to express a main property of the corresponding ion which is not expressed in a previous model, and the convergence value of the objective function is drastically reduced.

On the other hand, in a section (B) where the number of ion properties is large, even if the number of ion properties continues to increase, no further expressible main properties are available, and the convergence value of the objective function is gradually reduced.

Referring to the tendency of the graph, by finding a boundary between the sections (A) and (B), that is, a point (C) at which the slope of the graph changes, an appropriate number of ion properties, that is, a number of ion properties that accurately reflect main property data may be determined.

Alternatively, as a more quantitative method, a method, such as the Bayesian information criterion (BIC) method or the Akaike information criterion (AIC) method, may be used. In the methods, by giving a penalty based on the complexity of a model, a reduction in the slope of the graph is prevented so that the number of ion properties may be clearly determined.

A method of constructing the prior information matrix Y is described in detail below.

<<Construction of prior information matrix Y>>

Prior information on an ion according to an example embodiment may include an ionic radius and an oxidation number. This is based on Goldberg's law that ion substitution of a crystal structure is enabled when two ions have similar or the same ionic radii and no significant difference in oxidation number.

Meanwhile, both an oxidation number and an ionic radius are not accurately determined values but are values distributed in a certain range. In particular, in many cases, an ionic radius is not measured for a particular oxidation number.

For example, the prior information data may be expressed as a distribution curve p(s) as shown in FIG. 7.

To construct a matrix from prior information which has a particular distribution in consecutive sections, the amount of ions distributed in a particular section may be used.

For example, the amount of i-th ions distributed in a j-th range group may be calculated, and the calculated value may be set as the value of (i, j) element of the prior information matrix Y.

Here, each element value may be expressed as an integral value of a distribution in the corresponding section as the following expression:

[Expression 17]

$$Y_{ij} = \int_{Bi}^{Ei} p_i(s)\,ds.$$

Here, $p_i(s)$ denotes a distribution of prior information s on the i-th ion, and Bi and Ei denote a start point and an end point of a j-th property section, respectively.

For example, when a distribution curve p(s) shown in FIG. 7 shows a distribution of radius data of the i-th ion and a range of an ionic radius is divided into four sections (a section from a zeroth radius to a first radius, a section from the first radius to a second radius, a section from the second radius to a third radius, and a section from the third radius to a fourth radius), an (i, 1) element of the prior information matrix Y may be a value obtained by integrating the distribution curve p(s) from the zeroth radius to the first radius.

Also, an (i, 2) element of the prior information matrix Y may be a value obtained by integrating the distribution curve p(s) from the first radius to the second radius, an (i, 3) element of the prior information matrix Y may be a value obtained by integrating the distribution curve p(s) from the second radius to the third radius, and an (i, 4) element of the prior information matrix Y may be a value obtained by integrating the distribution curve p(s) from the third radius to the fourth radius.

A method of determining the distribution $p_i(s)$ of prior information is described in detail below.

<<Determination of basic prior information>>

When prior information is an oxidation number, a normal distribution which has a certain variance with respect to the known oxidation number may be used.

For example, when the oxidation number of an ion is +2, a normal distribution with an average of +2 and a standard deviation of 1 may be used as a distribution for the oxidation number of the ion. For example, the value of the standard deviation may be determined on the basis of known prior knowledge.

Meanwhile, when prior information is about ionic radius, ionic radii for all possible oxidation numbers may not be measured, and an unknown radius value may be present.

In addition, according to a crystal structure, the oxidation number of an ion may not be an integer but may be a real number. In this case, there may be no information on the ionic radius of the ion having an oxidation number which is a real number.

When known information is limited as above, the final distribution of prior information values may be determined through regression analysis.

<<Determination of distribution of prior information through probabilistic regression analysis model>>

A principle where the larger the oxidation number of an ion, the smaller the radius of the ion may hold. This is because, as the oxidation number increases, the number of electrons included in the ion is reduced, and the ionic radius decreases.

This relationship may be expressed by using a simple linear model. By probabilistically modeling the relationship and calculating a post probability, a distribution of ionic radius values for an arbitrary oxidation number may be determined. First, a likelihood of a known ionic radius value t may be expressed by using a normal distribution as follows:

[Expression 18]

$$p(\mathbf{t}|\mathbf{X}, \mathbf{w}, \beta) = \prod_{i=1}^{M} \mathbb{N}(t_i|\mathbf{w}^T\mathbf{x_i}, \beta^{-1}).$$

Here, X is a matrix including input values, such as a given oxidation number, etc., and a vector w is a parameter representing a linear relationship between an ionic radius and an oxidation number. $\beta$ is a parameter indicating the error size of a model. $\mathbb{N}(x|a,b)$ denotes a value corresponding to x in a normal distribution with an average of a and a variance of b. Also, a prior probability relative to the parameter w, which is desired to be calculated, may be expressed as the following normal distribution:

[Expression 19]

$$p(w) = \mathbb{N}(w|m_0, S_0)$$

Here, $m_0$ and $S_0$ are initial values of an average and a variance given to the parameter, respectively. When there is no other information, a zero vector and an identity matrix are used as $m_0$ and $S_0$.

Meanwhile, when the post probability is calculated from the likelihood and the prior probability according to Bayesian theory, the post probability may be expressed as follows:

[Expression 20]

$$p(w|t) = \mathbb{N}(w|m_N, S_N),$$
$$m_N = S_N(S_0^{-1}m_0 + \beta X^T t)$$
$$S_N = (S_0^{-1} + \beta X^T X)$$

By applying the above equations to a known ionic radius, it is possible to calculate a distribution of radius values for an arbitrary oxidation number of a target ion. A detailed calculation of the distribution may be made by calculating a predictive probability distribution on the basis of the post probability, and the predictive probability distribution may be expressed as a normal distribution as follows:

[Expression 21]

$$p(t|t, X, \beta) = \mathbb{N}\left(t|m_N^T x, \beta^{-1} + x^T S_N x\right).$$

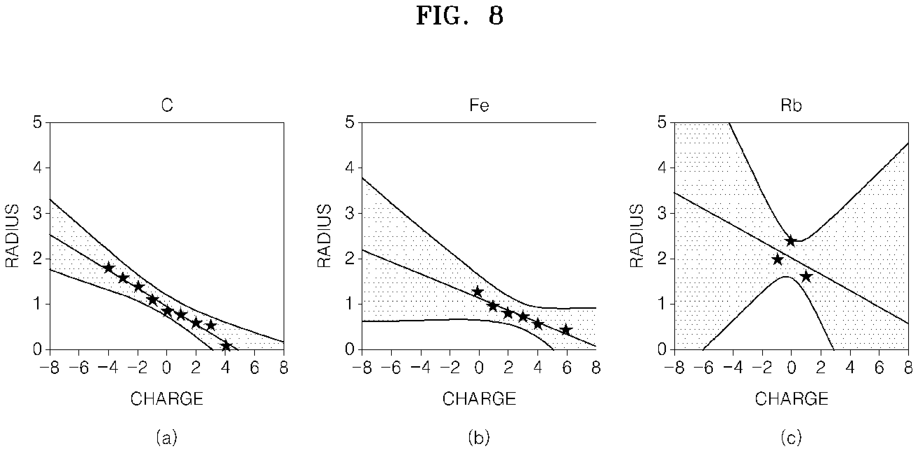

FIG. 8 shows graphs of distributions of radius values of C, Fe, and Rb relative to oxidation numbers according to the above expression.

Referring to the graphs, the illustrated points shown in the graphs denote known valence-specific ionic radius data, and the linear straight lines denote distributions of ionic radii calculated for arbitrary oxidation numbers by using the data. Shaded areas denote standard deviations. The standard deviations in FIG. 8 are shown to be narrow in a predictable portion and wide in an unpredictable portion.

A system and method for searching for a new material according to example embodiments are not limited to constitutions and methods of the above-described example embodiments, and all or some of the example embodiments may be selectively combined so that various modifications may be made.

As described above, according to the one or more of the above example embodiments, an unknown ion substitution tendency between ions may be predicted, and thus the reliability of results of a search for a new material may be increased.

Also, by using prior information data, it is possible to ensure the diversity of new materials groups, and information accumulated during a research process may be used for the development of a new material in an integrated manner.

For example, after calculating the probability of substitution of a plurality of candidate new materials, it may be possible to synthesize those candidate new materials having a higher rather than a lower probability. In particular, of the plurality of candidate new materials, it may be suitable to synthesize some or all of those candidate new materials having one of the top N probabilities, where N is an integer between 0 and 10. It may also be suitable to synthesize those candidate new materials where the probability is greater than a predetermined threshold probability.

Example embodiments allow for the prediction of new and yet-unknown material structures based on the high probability of ion substitution in a crystal structure corresponding to the new materials. As a result, new material structures may be discovered without having to undergo lengthy and expensive trial and error.

According to example embodiments, in the case of materials that are typically difficult and expensive to manufacture such as, e.g., drugs, cosmetics, carbon fiber, batteries, semiconductors, and the like, example embodiments provide methods of conceiving new material structures. For example, a Si-Ge (Group IV) semiconductor arranged in a diamond crystalline configuration and including one or more dopants may be predicted based on the probability of ion substitution of a dopant. Alternatively, Group III and Group V semiconductors forming a cubic-crystal configuration may also be predicted based on the ion substitution tendency of the atoms constituting the crystal, or on the ion substitution tendency of dopants.

In addition, other example embodiments can also be implemented through computer readable code/instructions in/on a medium, e.g., a computer readable medium, to control at least one processing element to implement any above described example embodiment. The medium can correspond to any medium/media permitting the storage and/or transmission of the computer readable code.

The computer readable code can be recorded/transferred on a medium in a variety of ways, with examples of the medium including recording media, such as magnetic storage media (e.g., ROM, floppy disks, hard disks, etc.) and optical recording media (e.g., CD-ROMs, or DVDs), and transmission media such as Internet transmission media. Thus, the medium may be such a defined and measurable structure including or carrying a signal or information, such as a device carrying a bitstream according to one or more example embodiments. The media may also be a distributed network, so that the computer readable code is stored/transferred and executed in a distributed fashion. Furthermore, the processing element could include a processor or a computer processor, and processing elements may be distributed and/or included in a single device.

It should be understood that the example embodiments described therein should be considered in a descriptive sense only and not for purposes of limitation. Descriptions of features within each example embodiment should typically be considered as available for other similar or the same features in other example embodiments.

**Claims**

1. A method of searching for a new material using computer readable code on a computer readable medium to control at least one processing element to implement the method, the method comprising:

    determining (120, S410) a substitution tendency matrix X including substitution tendency data of ions based on existing crystal structure data;

calculating (140, S430, S530) an ion property matrix U by applying a symmetric matrix factorization model to the substitution tendency matrix X, wherein the symmetric matrix factorization model is $\mathbf{X} \approx \mathbf{U}\mathbf{U}^T$;

determining (130, S440) substitution tendency prediction data based on the calculated ion property matrix U; and calculating (150, 160, S450, S550) a measure of the probability of there being new crystal structures by calculating probabilities of substitution of ions of the new crystal structures into the existing crystal structure data based on the substitution tendency prediction data;

wherein the substitution tendency matrix X contains substitution tendency vectors x each having N components, each of the N components being a value denoting a substitution tendency of any one ion for one of N ions;

wherein when $x_i$ denotes a substitution tendency vector of an i-th ion among the N ions, a first component x(1i) of $x_i$ is a value denoting a substitution tendency of the i-th ion for a first ion, and a second component x(2i) of $x_i$ is a value denoting a substitution tendency of the i-th ion for a second ion, and so on, and when a substitution tendency of the i-th ion for a j-th ion is unknown, a j-th component x(ji) of $x_i$ is set to 0;

wherein the substitution tendency vector $x_i$ may be expressed as a linear combination of several ion property vectors ($u_1$, $u_2$, ..., and $u_R$) as follows:

[Expression 3]

$$\mathbf{x}_i \approx a_{1i} \times \mathbf{u}_1 + a_{2i} \times \mathbf{u}_2 + a_{3i} \times \mathbf{u}_3 + \cdots + a_{Ri} \times \mathbf{u}_R,$$

wherein properties of the ion are classified into R types, and each $\mathbf{u}_j$ is a vector denoting a j-th property among the R property types;

wherein each value of $a_{ji}$ denotes how important the j-th ion property vector is in expressing a substitution tendency of the i-th ion, such that each value of $a_{ji}$ may be a weight indicating how much of the j-th property the i-th ion has;

whereby the substitution tendency vector may be expressed with a number R of ion property vectors that is smaller than a number of existing ion property vectors, obtained by condensing the properties of the ion;

wherein the substitution tendency vector $x_i$ of the i-th ion may be expressed as follows:

[Expression 4]

$$\mathbf{x}_i \approx \mathbf{U}\mathbf{a}_i,$$

in which U is an ion property matrix including ion property vectors $u_1$, $u_2$, ..., $u_R$ of Expression 3, rows denoting ions, and columns denoting properties of the ions, wherein the size of the ion property matrix U is N*R,

and in which $a_i$ is a weight vector having R components, which are the weights $a_{1i}$, $a_{2i}$, ..., $a_{Ri}$ of Expression 3 above;

wherein by applying a basic matrix factorization model to the substitution tendency matrix X, the substitution tendency matrix X may be expressed as follows:

[Expression 5]

$$\mathbf{X} \approx \mathbf{U}\mathbf{A}^T,$$

in which A is a weight matrix including the weight vectors for the N ions, the size of the weight matrix A being N*R;

and in which $\mathbf{A}^T$ denotes a transpose matrix of the weight matrix A, which is obtained by interchanging rows and columns of the weight matrix A;

wherein the substitution tendency matrix X, in which both rows and columns denote ions and are symmetrical to each other, may be expressed by using the ion property matrix U as follows:

[Expression 6]

$$\mathbf{X} \approx \mathbf{U}\mathbf{U}^T,$$

wherein optimization is performed to minimize a difference **X-UU$^T$** between the substitution tendency matrix X and the matrix factorization model **UU$^T$** so that the ion property matrix U is determined.

2. The method of claim 1, wherein the calculating of the ion property matrix U further comprises assigning a weight matrix W to **X - UU$^T$** to exclude data indicating that a substitution tendency is unknown from elements of the substitution tendency matrix X during a process of the calculating.

3. The method of claim 1 or 2, further comprising acquiring (S510) a prior information matrix Y including prior information data,
   wherein the calculating of the ion property matrix U comprises calculating the ion property matrix U based on the substitution tendency matrix X and the prior information matrix Y.

4. The method of claim 3, wherein the prior information data includes at least one of oxidation number data and radius data.

5. The method of any preceding claim, further comprising obtaining (160) an existing crystal structure from the existing crystal structure data (110) and generating (160) the new predicted crystal structures (PREDICTED STRUCTURE) in order of the probabilities of substitution of the new crystal structures.

6. A system for searching for a new material, comprising:

   a substitution tendency extractor (120) configured to calculate a substitution tendency matrix X including substitution tendency data based on existing crystal structure data;
   a substitution tendency predictor (130) configured to calculate an ion property matrix U by applying a symmetric matrix factorization model to the substitution tendency matrix X, and configured to acquire substitution tendency prediction data based on the ion property matrix U wherein the symmetric matrix factorization model for the substitution tendency matrix X is $\mathbf{x} \approx \mathbf{UU^T}$; and
   a substitution probability model builder (150) configured to calculate a measure of the probability of there being new materials having respective new crystal structures by calculating probabilities of substitution of ions of the new crystal structures into the existing crystal structure data based on the substitution tendency prediction data;
   wherein the substitution tendency matrix X contains substitution tendency vectors x each having N components, each of the N components being a value denoting a substitution tendency of any one ion for one of N ions;
   wherein when $x_i$ denotes a substitution tendency vector of an i-th ion among the N ions, a first component x(1i) of $x_i$ is a value denoting a substitution tendency of the i-th ion for a first ion, and a second component x(2i) of $x_i$ is a value denoting a substitution tendency of the i-th ion for a second ion, and so on, and when a substitution tendency of the i-th ion for a j-th ion is unknown, a j-th component x(ji) of $x_i$ is set to 0;
   wherein the substitution tendency vector $x_i$ may be expressed as a linear combination of several ion property vectors ($u_1$, $u_2$, ..., and $u_R$) as follows:

   [Expression 3]

   $$\mathbf{x}_i \approx a_{1i} \times \mathbf{u}_1 + a_{2i} \times \mathbf{u}_2 + a_{3i} \times \mathbf{u}_3 + \cdots + a_{Ri} \times \mathbf{u}_R,$$

   wherein properties of the ion are classified into R types, and each $\mathbf{u_j}$ is a vector denoting a j-th property among the R property types;
   wherein each value of $a_{ji}$ denotes how important the j-th ion property vector is in expressing a substitution tendency of the i-th ion, such that each value of $a_{ji}$ may be a weight indicating how much of the j-th property the i-th ion has;
   whereby the substitution tendency vector may be expressed with a number R of ion property vectors that is smaller than a number of existing ion property vectors, obtained by condensing the properties of the ion;
   wherein the substitution tendency vector $x_i$ of the i-th ion may be expressed as follows:

   [Expression 4]

   $$\mathbf{x}_i \approx \mathbf{Ua}_{i,}$$

in which U is an ion property matrix including ion property vectors $u_1$, $u_2$, ..., $u_R$ of Expression 3, rows denoting ions, and columns denoting properties of the ions, wherein the size of the ion property matrix U is N*R,

and in which $a_i$ is a weight vector having R components, which are the weights $a_{1i}$, $a_{2i}$, ..., $a_{Ri}$ of Expression 3 above;

wherein by applying a basic matrix factorization model to the substitution tendency matrix X, the substitution tendency matrix X may be expressed as follows:

[Expression 5]

$$\mathbf{X} \approx \mathbf{U}\mathbf{A}^{\mathrm{T}},$$

in which A is a weight matrix including the weight vectors for the N ions, the size of the weight matrix A being N*R;

and in which $\mathbf{A}^{\mathrm{T}}$ denotes a transpose matrix of the weight matrix A, which is obtained by interchanging rows and columns of the weight matrix A;

wherein the substitution tendency matrix X, in which both rows and columns denote ions and are symmetrical to each other, may be expressed by using the ion property matrix U as follows:

[Expression 6]

$$\mathbf{X} \approx \mathbf{U}\mathbf{U}^{\mathrm{T}},$$

wherein the system is configured to perform optimization to minimize a difference $\mathbf{X} - \mathbf{U}\mathbf{U}^{\mathrm{T}}$ between the substitution tendency matrix X and the matrix factorization model $\mathbf{U}\mathbf{U}^{\mathrm{T}}$ so as to determine the ion property matrix U.

7. The system of claim 6, wherein the substitution tendency extractor (120) is configured to assign a weight matrix W to $\mathbf{X} - \mathbf{U}\mathbf{U}^{\mathrm{T}}$ to exclude data indicating that a substitution tendency is unknown from elements of the substitution tendency matrix X during a process of the calculation.

8. The system of claim 6 or 7, further comprising a prior information model builder (140) configured to calculate a prior information matrix Y including prior information data,
   wherein the substitution tendency extractor (120) is configured to calculate the ion property matrix U based on the substitution tendency matrix X and the prior information matrix Y.

9. The system of claim 8, wherein the substitution tendency extractor (120) is configured to calculate the ion property matrix U by applying a matrix co-factorization model, which causes the ion property matrix U to be included in the substitution tendency matrix X and the prior information matrix Y in common, to the substitution tendency matrix X and the prior information matrix Y.

10. The system of claim 8 or 9, wherein the substitution tendency extractor (120) is configured to calculate the ion property matrix U under a constraint condition where element values of the ion property matrix U are not negative numbers.

11. The system of claim 8, 9 or 10, wherein the prior information data includes at least one of oxidation number data and radius data.

12. The system of any of claims 6 to 11, further comprising a new crystal structure predictor (160) that is configured to generate new predicted crystal structures in order of the probabilities of substitution of the new crystal structures.

**Patentansprüche**

1. Verfahren zum Suchen nach einem neuen Material unter Verwendung von computerlesbarem Code auf einem computerlesbaren Medium, um mindestens ein Verarbeitungselement zu steuern, um das Verfahren zu implementieren, wobei das Verfahren folgendes umfasst:

Bestimmen (120, S410) einer Substitutionstendenzmatrix X, die Substitutionstendenzdaten von Ionen auf der Basis bestehender Kristallstrukturdaten aufweist;

Berechnen (140, S430, S530) einer Ioneneigenschaftsmatrix U durch Anwenden eines symmetrischen Matrixfaktorisierungsmodells auf die Substitutionstendenzmatrix X, wobei das symmetrische Matrixfaktorisierungsmodell $X \approx UU^T$ ist;

Bestimmen (130, S440) von Substitutionstendenz-Prädiktionsdaten auf der Basis der berechneten Ioneneigenschaftsmatrix U; und

Berechnen (150, 160, S450, S550) einer Messgröße der Wahrscheinlichkeit für das Vorhandensein neuer Kristallstrukturen durch Berechnen von Wahrscheinlichkeiten der Substitution von Ionen der neuen Kristallstrukturen in die bestehenden Kristallstrukturdaten auf der Basis der Substitutionstendenz-Prädiktionsdaten; wobei die Substitutionstendenzmatrix X Substitutionstendenzvektoren x enthält, die jeweils N Komponenten aufweisen, wobei jede der N Komponenten ein Wert ist, der eine Substitutionstendenz für ein beliebiges Ion für eines von N Ionen bezeichnet;

wobei, wenn $x_i$ einen Substitutionstendenzvektor eines i-ten Ions der N Ionen bezeichnet, eine erste Komponente x(1i) von $x_i$ ein Wert ist, der eine Substitutionstendenz des i-ten Ions für ein erstes Ion bezeichnet, und wobei eine zweite Komponente x(2i) von $x_i$ ein Wert ist, der eine Substitutionstendenz des i-ten Ions für ein zweites Ion bezeichnet, und so weiter, und wobei, wenn eine Substitutionstendenz des i-ten Ions für ein j-tes Ion unbekannt ist, eine j-te Komponente x(ji) von $x_i$ auf 0 gesetzt wird;

wobei der Substitutionstendenzvektor $x_i$ als eine lineare Kombination mehrerer Ioneneigenschaftsvektoren ($u_1$, $u_2$, ... und $u_R$) wie folgt ausgedrückt werden kann:

[Ausdruck 3]

$$x_i \approx a_{1i} \times u_1 + a_{2i} \times u_2 + a_{3i} \times u_3 + ... + a_{Ri} \times u_R,$$

wobei die Eigenschaften des Ions in R Typen klassifiziert sind, und wobei jedes $u_j$ ein Vektor ist, der eine j-te Eigenschaft der R Eigenschaftstypen bezeichnet;

wobei jeder Wert von $a_{ji}$ bezeichnet, wie wichtig der j-te Eigenschaftsvektor ist, um eine Substitutionstendenz des i-ten Ions auszudrücken, so dass jeder Wert von $a_{ji}$ eine Gewichtung sein kann, die anzeigt, welche Stärke der j-ten Eigenschaft das i-te Ion aufweist;

wobei der Substitutionstendenzvektor mit einer Anzahl R von Ioneneigenschaftsvektoren ausgedrückt werden kann, die kleiner ist als eine Anzahl bestehender Ioneneigenschaftsvektoren, die durch Kondensieren der Eigenschaften des Ions erhalten werden;

wobei der Substitutionstendenzvektor $x_i$ des i-ten Ions wie folgt ausgedrückt werden kann:

[Ausdruck 4]

$$X_i \approx Ua_i,$$

wobei U eine Ioneneigenschaftsmatrix ist, die Ioneneigenschaftsvektoren $u_1$, $u_2$, ..., $u_R$ von Ausdruck 3 aufweist, wobei Zeilen Ionen bezeichnen und Spalten Eigenschaften der Ionen bezeichnen, wobei die Größe der Ioneneigenschaftsmatrix U N*R ist,

und wobei $a_i$ ein Gewichtungsvektor ist, der R Komponenten aufweist, bei denen es sich um die Gewichtungen $a_{1i}$, $a_{2i}$, ..., $a_{Ri}$ aus Ausdruck 3 oben handelt;

wobei durch Anwenden eines grundlegenden Matrixfaktorisierungsmodells auf die Substitutionstendenzmatrix X die Substitutionstendenzmatrix X wie folgt ausgedrückt werden kann:

[Ausdruck 5]

$$X \approx UA^T,$$

wobei A eine Gewichtungsmatrix ist, welche die Gewichtungsfaktoren für die N Ionen aufweist, wobei die Größe der Gewichtungsmatrix N*R ist;

und wobei $A^T$ eine transponierte Matrix der Gewichtungsmatrix A ist, die erhalten wird durch Austauschen der Zeilen und Spalten der Gewichtungsmatrix A;

wobei die Substitutionsmatrix X, in welcher sowohl die Zeilen als auch die Spalten Ionen bezeichnen und symmetrisch zueinander sind, unter Verwendung der Ioneneigenschaftsmatrix U wie folgt ausgedrückt werden kann:

[Ausdruck 6]

$$X \approx UU^T,$$

wobei eine Optimierung durchgeführt wird, um eine Differenz $X - UU^T$ zwischen der Substitutionstendenzmatrix X und dem Matrixfaktorisierungsmodell $UU^T$ zu minimieren, so dass die Ioneneigenschaftsmatrix U bestimmt wird.

2. Verfahren nach Anspruch 1, wobei das Berechnen der Ioneneigenschaftsmatrix U ferner das Zuweisen einer Gewichtungsmatrix W zu $X - UU^T$ umfasst, um Daten auszuschließen, die anzeigen, dass eine Substitutionstendenz aus den Elementen der Substitutionstendenzmatrix X während einem Prozess des Berechnens unbekannt ist.

3. Verfahren nach Anspruch 1 oder 2, ferner umfassend das Erfassen (S510) einer früheren Informationsmatrix Y, die frühere Informationsdaten aufweist,
wobei das Berechnen der Ioneneigenschaftsmatrix U das Berechnen der Ioneneigenschaftsmatrix U auf der Basis der Substitutionstendenzmatrix X und der früheren Informationsmatrix Y umfasst.

4. Verfahren nach Anspruch 3, wobei die früheren Informationsdaten wenigstens eines der folgenden aufweisen: Oxidationsstufendaten und Radiusdaten.

5. Verfahren nach einem der vorstehenden Ansprüche, ferner umfassend das Ermitteln (160) einer bestehenden Kristallstruktur aus den bestehenden Kristallstrukturdaten (110) und das Erzeugen (160) der neuen prädizierten Kristallstrukturen (PRÄDIZIERTE STRUKTUR) in der Reihenfolge der Wahrscheinlichkeiten der Substitution der neuen Kristallstrukturen.

6. System zum Suchen nach einem neuen Material, umfassend:

einen Substitutionstendenzextraktor (120), der so gestaltet ist, dass er eine Substitutionstendenzmatrix X berechnet, die Substitutionstendenzdaten auf der Basis bestehender Kristallstrukturdaten aufweist;
einen Substitutionstendenzprädiktor (130), der so gestaltet ist, dass er eine Ioneneigenschaftsmatrix U durch Anwenden eines symmetrischen Matrixfaktorisierungsmodells auf die Substitutionstendenzmatrix X berechnet, und wobei er so gestaltet ist, dass er Substitutionstendenz-Prädiktionsdaten auf der Basis der Ioneneigenschaftsmatrix U erfasst, wobei das symmetrische Matrixfaktorisierungsmodell für die Substitutionstendenzmatrix X gleich $X \approx UU^T$ ist; und
einen Substitutionswahrscheinlichkeitsmodellgestalter (150), der so gestaltet ist, dass er eine Messgröße der Wahrscheinlichkeit für das Vorhandensein neuer Materialien mit entsprechenden neuen Kristallstrukturen durch Berechnen von Wahrscheinlichkeiten der Substitution von Ionen der neuen Kristallstrukturen in die bestehenden Kristallstrukturdaten auf der Basis der Substitutionstendenz-Prädiktionsdaten berechnet;
wobei die Substitutionstendenzmatrix X Substitutionstendenzvektoren x enthält, die jeweils N Komponenten aufweisen, wobei jede der N Komponenten ein Wert ist, der eine Substitutionstendenz für ein beliebiges Ion für eines von N Ionen bezeichnet;
wobei, wenn $x_i$ einen Substitutionstendenzvektor eines i-ten Ions der N Ionen bezeichnet, eine erste Komponente $x(1i)$ von $x_i$ ein Wert ist, der eine Substitutionstendenz des i-ten Ions für ein erstes Ion bezeichnet, und wobei eine zweite Komponente $x(2i)$ von $x_i$ ein Wert ist, der eine Substitutionstendenz des i-ten Ions für ein zweites Ion bezeichnet, und so weiter, und wobei, wenn eine Substitutionstendenz des i-ten Ions für ein j-tes Ion unbekannt ist, eine j-te Komponente $x(ji)$ von $x_i$ auf 0 gesetzt wird;
wobei der Substitutionstendenzvektor $x_i$ als eine lineare Kombination mehrerer Ioneneigenschaftsvektoren ($u_1$, $u_2$, ... und $u_R$) wie folgt ausgedrückt werden kann:

[Ausdruck 3]

$$x_i \approx a_{1i} \times u_1 + a_{2i} \times u_2 + a_{3i} \times u_3 + \ldots + a_{Ri} \times u_R,$$

wobei die Eigenschaften des Ions in R Typen klassifiziert sind, und wobei jedes $u_j$ ein Vektor ist, der eine j-te Eigenschaft der R Eigenschaftstypen bezeichnet;

wobei jeder Wert von $a_{ji}$ bezeichnet, wie wichtig der j-te Eigenschaftsvektor ist, um eine Substitutionstendenz des i-ten Ions auszudrücken, so dass jeder Wert von $a_{ji}$ eine Gewichtung sein kann, die anzeigt, welche Stärke der j-ten Eigenschaft das i-te Ion aufweist;

wobei der Substitutionstendenzvektor mit einer Anzahl R von Ioneneigenschaftsvektoren ausgedrückt werden kann, die kleiner ist als eine Anzahl bestehender Ioneneigenschaftsvektoren, die durch Kondensieren der Eigenschaften des Ions erhalten werden;

wobei der Substitutionstendenzvektor $x_i$ des i-ten Ions wie folgt ausgedrückt werden kann:

[Ausdruck 4]

$$X_i \approx U a_i,$$

wobei U eine Ioneneigenschaftsmatrix ist, die Ioneneigenschaftsvektoren $u_1$, $u_2$, ..., $u_R$ von Ausdruck 3 aufweist, wobei Zeilen Ionen bezeichnen und Spalten Eigenschaften der Ionen bezeichnen, wobei die Größe der Ioneneigenschaftsmatrix U N*R ist,

und wobei $a_i$ ein Gewichtungsvektor ist, der R Komponenten aufweist, bei denen es sich um die Gewichtungen $a_{1i}$, $a_{2i}$, ..., $a_{Ri}$ aus Ausdruck 3 oben handelt;

wobei durch Anwenden eines grundlegenden Matrixfaktorisierungsmodells auf die Substitutionstendenzmatrix X die Substitutionstendenzmatrix X wie folgt ausgedrückt werden kann:

[Ausdruck 5]

$$X \approx U A^T,$$

wobei A eine Gewichtungsmatrix ist, welche die Gewichtungsfaktoren für die N Ionen aufweist, wobei die Größe der Gewichtungsmatrix N*R ist;

und wobei $A^T$ eine transponierte Matrix der Gewichtungsmatrix A ist, die erhalten wird durch Austauschen der Zeilen und Spalten der Gewichtungsmatrix A;

wobei die Substitutionsmatrix X, in welcher sowohl die Zeilen als auch die Spalten Ionen bezeichnen und symmetrisch zueinander sind, unter Verwendung der Ioneneigenschaftsmatrix U wie folgt ausgedrückt werden kann:

[Ausdruck 6]

$$X \approx U U^T,$$

wobei das System für die Durchführung einer Optimierung gestaltet ist, um eine Differenz $X - U U^T$ zwischen der Substitutionstendenzmatrix X und dem Matrixfaktorisierungsmodell $U U^T$ zu minimieren, so dass die Ioneneigenschaftsmatrix U bestimmt wird.

7. System nach Anspruch 6, wobei der Substitutionstendenzextraktor (120) so gestaltet ist, dass er $X - U U^T$ eine Gewichtungsmatrix W zuweist, um Daten auszuschließen, die anzeigen, dass eine Substitutionstendenz aus den Elementen der Substitutionstendenzmatrix X während einem Prozess des Berechnens unbekannt ist.

8. System nach Anspruch 6 oder 7, ferner umfassend einen früheren Informationsmodellgestalter (140), der so gestaltet ist, dass er eine frühere Informationsmatrix Y berechnet, die frühere Informationsdaten aufweist; wobei der Substitutionstendenzextraktor (120) so gestaltet ist, dass er die Ioneneigenschaftsmatrix U auf der Basis der Substitutionstendenzmatrix X und der früheren Informationsmatrix Y berechnet.

9. System nach Anspruch 8, wobei der Substitutionstendenzextraktor (120) so gestaltet ist, dass er die Ioneneigenschaftsmatrix U berechnet durch Anwenden eines Matrixcofaktorisierungsmodells, das bewirkt, dass die Ioneneigenschaftsmatrix U in der Substitutionstendenzmatrix X als auch in der früheren Informationsmatrix Y enthalten ist, auf die Substitutionstendenzmatrix X und die frühere Informationsmatrix Y.

10. System nach Anspruch 8 oder 9, wobei der Substitutionstendenzextraktor (120) so gestaltet ist, dass er die Ioneneigenschaftsmatrix unter einer Zwangsbedingung berechnet, wobei die Elementwerte der Ioneneigenschaftsmatrix U keine negativen Zahlen sind.

11. System nach Anspruch 8, 9 oder 10, wobei die früheren Informationsdaten wenigstens eines der folgenden aufweisen: Oxidationsstufendaten und Radiusdaten.

12. System nach einem der Ansprüche 6 bis 11, ferner umfassend einen Prädiktor (160) für neue Kristallstrukturen, der so gestaltet ist, dass er neue prädizierte Kristallstrukturen in der Reihenfolge der Wahrscheinlichkeiten der Substitution der neuen Kristallstrukturen erzeugt.

**Revendications**

1. Procédé de recherche d'un nouveau matériau à l'aide d'un code lisible par ordinateur sur un support lisible par ordinateur pour commander au moins un élément de traitement afin de mettre en oeuvre le procédé, le procédé comprenant :

   la détermination (120, S410) d'une matrice de tendance à la substitution X comprenant des données de tendance à la substitution d'ions sur la base de données de structure cristalline existante ;
   le calcul (140, S430, S530) d'une matrice de propriétés d'ions U en appliquant un modèle de factorisation de matrice symétrique à la matrice de tendance à la substitution X, ledit modèle de factorisation de matrice symétrique étant $X \approx UU^T$ ;
   la détermination (130, S440) des données de prédiction de tendance à la substitution sur la base de la matrice de propriétés d'ions U calculée ;
   et le calcul (150, 160, S450, S550) d'une mesure de la probabilité de l'existence de nouvelles structures cristallines en calculant des probabilités de substitution des ions des nouvelles structures cristallines dans les données de structure cristalline existante sur la base des données de prédiction de tendance à la substitution ;
   ladite matrice de tendance à la substitution X contenant des vecteurs de tendance à la substitution x possédant chacun N composantes, chacune des N composantes étant une valeur dénotant une tendance à la substitution d'un ion quelconque pour l'un des N ions ;
   lorsque $x_i$ dénote un vecteur de tendance à la substitution d'un i-ème ion parmi les N ions, une première composante $x(1i)$ de $x_i$ étant une valeur dénotant une tendance à la substitution du i-ème ion pour un premier ion, et une seconde composante $x(2i)$ de $x_i$ étant une valeur dénotant une tendance à la substitution du i-ème ion pour un second ion, et ainsi de suite, et lorsqu'une tendance à la substitution du i-ème ion pour un j-ème ion est inconnue, une j-ème composante $x(ji)$ de $x_i$ étant fixée à 0 ;
   ledit vecteur de tendance à la substitution $x_i$ pouvant être exprimé sous la forme d'une combinaison linéaire de plusieurs vecteurs de propriété d'ions ($u_1$, $u_2$,.... et $u_R$) comme suit :

   [Expression 3]

   $$x_i \approx a_{1i} \times u_1 + a_{2i} \times u_2 + a_{3i} \times u_3 + \cdots + a_{Ri} \times u_R$$

   lesdites propriétés de l'ion étant classées en des types R, et chaque $u_j$ étant un vecteur dénotant une j-ème propriété parmi les types de propriétés R ;
   chaque valeur de $a_{ji}$ dénotant l'importance du vecteur de propriété du j-ième ion dans l'expression d'une tendance à la substitution du i-ème ion, de sorte que chaque valeur de $a_{ji}$ puisse être une pondération indiquant dans quelle proportion le i-ème ion possède la j-ème propriété ;
   moyennant quoi le vecteur de tendance à la substitution peut être exprimé avec un nombre R de vecteurs de propriété d'ions inférieur à un nombre de vecteurs de propriété d'ions existants, obtenus par condensation des propriétés de l'ion ;
   ledit vecteur de tendance à la substitution x; du i-ème ion pouvant être exprimé comme suit :

[Expression 4]

$$\mathbf{x_i} \approx \mathbf{U a_{i,}} \qquad \text{lequel U est une mat}$$

dans lequel U est une matrice de propriétés d'ions comprenant les vecteurs de propriétés d'ions $u_1$, $u_2$, ..., $u_R$ de l'expression 3, des lignes dénotant les ions et des colonnes dénotant les propriétés des ions, ladite taille de la matrice de propriétés d'ions U étant N*R, et dans lequel $a_i$ est un vecteur de pondération possédant R composantes, qui sont les pondérations $a_{1i}$, $a_{2i}$, ..., $a_{Ri}$ de l'expression 3 ci-dessus ;

en appliquant un modèle de factorisation de matrice de base à la matrice de tendance à la substitution X, ladite matrice de tendance à la substitution X pouvant être exprimée comme suit :

[Expression 5]

$$\mathbf{X} \approx \mathbf{U A^T}, \ \text{dans}$$

dans lequel A est une matrice de pondération comprenant les vecteurs de pondération pour les N ions, la taille de la matrice de pondération A étant N*R ;

et dans lequel $\mathbf{A^T}$ dénote la matrice transposée de la matrice de pondération A, obtenue en échangeant les lignes et les colonnes de la matrice de pondération A ;

ladite matrice de tendance à la substitution X, dans laquelle les lignes et les colonnes dénotent des ions et sont symétriques les unes par rapport aux autres, pouvant être exprimée à l'aide de la matrice de propriété d'ions U comme suit :

[Expression 6]

$$\mathbf{X} \approx \mathbf{U U^T}, \ \text{ladite}$$

ladite optimisation étant effectuée pour minimiser une différence $\mathbf{X} - \mathbf{U U^T}$ entre la matrice de tendance à la substitution X et le modèle de factorisation de matrice $\mathbf{U U^T}$ afin que la matrice de propriété d'ions U soit déterminée.

2. Procédé selon la revendication 1, ledit calcul de la matrice de propriété d'ions U comprenant en outre l'attribution d'une matrice de pondération W à $\mathbf{X} - \mathbf{U U^T}$ pour exclure des données, indiquant qu'une tendance à la substitution est inconnue, des éléments de la matrice de tendance à la substitution X durant un processus du calcul.

3. Procédé selon la revendication 1 ou 2, comprenant en outre l'acquisition (S510) d'une matrice d'informations antérieures Y comprenant des données d'informations antérieures, ledit calcul de la matrice de propriété d'ions U comprenant le calcul de la matrice de propriétés d'ions U sur la base de la matrice de tendance à la substitution X et de la matrice d'informations antérieures Y.

4. Procédé selon la revendication 3, lesdites données d'informations antérieures comprenant au moins l'une des données de nombre d'oxydation et des données de rayon.

5. Procédé selon l'une quelconque des revendications précédentes, comprenant en outre l'obtention (160) d'une structure cristalline existante à partir des données de structure cristalline existantes (110) et la génération (160) des nouvelles structures cristallines prédites (STRUCTURE PRÉDITE) dans l'ordre des probabilités de substitution des nouvelles structures cristallines.

6. Système destiné à la recherche d'un nouveau matériau, comprenant :

un extracteur de tendance à la substitution (120) conçu pour calculer une matrice de tendance à la substitution X comprenant des données de tendance à la substitution sur la base des données de structure cristalline existantes ;

un prédicteur de tendance à la substitution (130) conçu pour calculer une matrice de propriété d'ions U en appliquant un modèle de factorisation de matrice symétrique à la matrice de tendance à la substitution X, et conçu pour acquérir des données de prédiction de tendance à la substitution sur la base de la matrice de propriété d'ions U dans laquelle le modèle de factorisation de matrice symétrique pour la matrice de tendance à la substitution X est $X \approx UU^T$ ;

et un constructeur de modèle de probabilité de substitution (150) conçu pour calculer une mesure de la probabilité de l'existence de nouveaux matériaux possédant de nouvelles structures cristallines respectives en calculant des probabilités de substitution d'ions des nouvelles structures cristallines dans les données de structure cristalline existantes sur la base des données de prédiction de tendance à la substitution ;

ladite matrice de tendance à la substitution X contenant des vecteurs de tendance à la substitution x possédant chacun N composantes, chacune des N composantes étant une valeur dénotant une tendance à la substitution d'un ion quelconque pour l'un des N ions ;

lorsque $x_i$ dénote un vecteur de tendance à la substitution d'un i-ème ion parmi les N ions, une première composante x(1i) de $x_i$ étant une valeur dénotant une tendance à la substitution du i-ème ion pour un premier ion, et une seconde composante x(2i) de $x_i$ étant une valeur dénotant une tendance à la substitution du i-ème ion pour un second ion, et ainsi de suite, et lorsqu'une tendance à la substitution du i-ème ion pour un j-ème ion est inconnue, une j-ème composante x(ji) de $x_i$ étant fixée à 0 ;

ledit vecteur de tendance à la substitution $x_i$ pouvant être exprimé sous la forme d'une combinaison linéaire de plusieurs vecteurs de propriété d'ions ($u_1$, $u_2$,.... et $u_R$) comme suit :

[Expression 3]

$$x_i \approx a_{1i} \times u_1 + a_{2i} \times u_2 + a_{3i} \times u_3 + \cdots + a_{Ri} \times u_R$$

lesdites propriétés de l'ion étant classées en R types, et chaque $u_j$ étant un vecteur dénotant une j-ème propriété parmi les R types de propriétés ;

chaque valeur de $a_{ji}$ dénotant l'importance du vecteur de propriété du j-ème ion dans l'expression d'une tendance à la substitution du i-ème ion, de sorte que chaque valeur de $a_{ji}$ puisse être une pondération indiquant dans quelle proportion le i-ème ion possède la j-ème propriété ;

moyennant quoi le vecteur de tendance à la substitution peut être exprimé avec un nombre R de vecteurs de propriété d'ions inférieur à un nombre de vecteurs de propriété d'ions existants, obtenus par condensation des propriétés de l'ion ;

ledit vecteur de tendance à la substitution x; du i-ème ion pouvant être exprimé comme suit :

[Expression 4]

$$x_i \approx Ua_{i.}$$

dans laquelle U est une matrice de propriétés d'ions comprenant les vecteurs de propriétés d'ions $u_1$, $u_2$, ..., $u_R$ de l'expression 3, des lignes dénotant les ions et des colonnes dénotant les propriétés des ions, ladite taille de la matrice de propriétés d'ions U étant N*R, et dans laquelle $a_i$ est un vecteur de pondération possédant R composantes, qui sont les pondérations $a_{1i}$, $a_{2i}$, ..., $a_{Ri}$ de l'expression 3 ci-dessus ;

en appliquant un modèle de factorisation de matrice de base à la matrice de tendance à la substitution X, ladite matrice de tendance à la substitution X pouvant être exprimée comme suit :

ution X, ladite m

[Expression 5]

$$X \approx UA^T,$$

dans laquelle A est une matrice de pondération comprenant les vecteurs de pondération des N ions, la taille de la matrice de pondération A étant N*R ;

et dans lequel $A^T$ dénote la matrice transposée de la matrice de pondération A, obtenue en échangeant les lignes et les colonnes de la matrice de pondération A ;

ladite matrice de tendance à la substitution X, dans laquelle les lignes et les colonnes dénotent des ions et sont symétriques les unes par rapport aux autres, pouvant être exprimée à l'aide de la matrice de propriété d'ions U comme suit :

~~des ions et~~

~~de la matrice de~~

ledit système étant conçu pour effectuer une optimisation afin de minimiser une différence $X-UU^{T}$ entre la matrice de tendance à la substitution X et le modèle de factorisation de matrice $UU^{T}$ de façon à déterminer la matrice de propriété d'ions U.

7. Système selon la revendication 6, ledit extracteur de tendance à la substitution (120) étant conçu pour attribuer une matrice de pondération W à $X-UU^{T}$ afin d'exclure des données, indiquant qu'une tendance à la substitution est inconnue, des éléments de la matrice de tendance à la substitution X durant un processus du calcul.

8. Système selon la revendication 6 ou 7, comprenant en outre un constructeur de modèle d'informations antérieures (140) conçu pour calculer une matrice d'informations antérieures Y comprenant des données d'informations antérieures, ledit extracteur de tendance à la substitution (120) étant conçu pour calculer la matrice de propriété d'ions U sur la base de la matrice de tendance à la substitution X et de la matrice d'informations antérieures Y.

9. Système selon la revendication 8, ledit extracteur de tendance à la substitution (120) étant conçu pour calculer la matrice de propriété d'ions U en appliquant un modèle de co-factorisation de matrice, qui amène la matrice de propriété d'ions U à être comprise dans la matrice de tendance à la substitution X et la matrice d'informations antérieures Y en commun, à la matrice de tendance à la substitution X et la matrice d'informations antérieures Y.

10. Système selon la revendication 8 ou 9, ledit extracteur de tendance à la substitution (120) étant conçu pour calculer la matrice de propriété d'ions U sous des conditions de contrainte où les valeurs d'élément de la matrice de propriété d'ions U ne sont pas des nombres négatifs.

11. Système selon la revendication 8, 9 ou 10, lesdites données d'informations antérieures comprenant au moins l'une des données de nombre d'oxydation et des données de rayon.

12. Système selon l'une quelconque des revendications 6 à 11, comprenant en outre un nouveau prédicteur de structure cristalline (160) qui est conçu pour générer de nouvelles structures cristallines prédites dans l'ordre des probabilités de substitution des nouvelles structures cristallines.

# FIG. 1

100

110
EXISTING CRYSTAL STRUCTURE DATABASE

120
SUBSTITUTION TENDENCY EXTRACTOR

EXISTING STRUCTURE

130
SUBSTITUTION TENDENCY PREDICTOR

140
PRIOR INFORMATION MODEL BUILDER

160
NEW CRYSTAL STRUCTURE PREDICTOR

150
SUBSTITUTION PROBABILITY MODEL BUILDER

PREDICTED STRUCTURE

# FIG. 2

CaTiO₃

(a)

BaTiO₃

(b)

# FIG. 3

PROPERTY OF ION

ION EXCHANGE TENDENCY

# FIG. 4

START

CALCULATE SUBSTITUTION
TENDENCY MATRIX X — S410

APPLY SYMMETRIC MATRIX
FACTORIZATION MODEL
TO MATRIX X — S420

CALCULATE ION PROPERTY
MATRIX U — S430

ACQUIRE SUBSTITUTION
TENDENCY PREDICTION DATA — S440

CALCULATE PROBABILITY OF
SUBSTITUTION OF NEW
CRYSTAL STRUCTURE — S450

FINISH

# FIG. 5

START

CALCULATE SUBSTITUTION
TENDENCY MATRIX X AND
PRIOR INFORMATION MATRIX Y — S510

APPLY CO-FACTORIZATION
MODEL — S520

CALCULATE ION PROPERTY
MATRIX U — S530

ACQUIRE SUBSTITUTION
TENDENCY PREDICTION DATA — S540

CALCULATE PROBABILITY OF
SUBSTITUTION IN NEW
CRYSTAL STRUCTURE — S550

FINISH

## FIG. 6

## FIG. 7

**FIG. 8**

**EP 2 881 874 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090157369 A